# EUROPEAN PATENT SPECIFICATION

(11) **EP 2 585 829 B1**
(45) Date of publication and mention of the grant of the patent: **10.12.2014**
(21) Application number: 11741265.0
(22) Date of filing: 28.06.2011
(51) Int. Cl.: G01N 33/487, H02J 7/00

(54) **HAND-HELD TEST METER WITH DEEP POWER CONSERVATION MODE**
HANDPRÜFZÄHLER MIT DEEP-POWER-BEIBEHALTUNGSMODUS
COMPTEUR DE TEST MANUEL POUVANT FONCTIONNER EN MODE PROFOND DE GESTION DE LA CONSOMMATION

(30) Priority: 18.01.2011 US 201113008405; 28.06.2010 US 359236 P
(43) Date of publication of application: 01.05.2013
(73) Proprietor: Cilag GmbH International, 6300 Zug (CH)
(72) Inventor: GUTHRIE, Brian, Inverness-shire IV2 6RG (GB); BORGHI, Tommaso, Milan (IT)
(74) Representative: Brunner, John Michael Owen
(86) International application number: PCT/GB2011/000974
(87) International publication number: WO 2012/001351

(56) References cited:
- WO-A1-00/05581
- WO-A1-2006/070200
- US-A- 6 072 250
- US-A1- 2009 076 359
- US-A1- 2009 264 337
- US-B1- 6 586 911

## Description

### RELATED APPLICATIONS

This application claims priority pursuant to 35 U.S.C. §119 to U.S. Provisional Patent Application Ser. No. 61/359,236, entitled "Hand-Held Test Meter With Deep Power Conservation Mode," filed on June 28, 2010.

### BACKGROUND OF THE INVENTION

### Field of the Invention

The present invention relates, in general, to medical devices and, in particular, to test meters and related methods.

### Description of Related Art

The determination (e.g., detection and/or concentration measurement) of an analyte in a fluid sample is of particular interest in the medical field. For example, it can be desirable to determine glucose, ketone bodies, cholesterol, lipoproteins, triglycerides, acetaminophen and/or HbA1c concentrations in a sample of a bodily fluid such as urine, blood, plasma or interstitial fluid. Such determinations can be achieved using a hand-held test meter in combination with analytical test strips (e.g., electrochemical-based analytical test strips).

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings, in which like numerals indicate like elements, of which:
FIG. 1 is a simplified top view of a hand-held test meter according to an embodiment of the present invention;
FIG. 2 is a simplified block diagram of various blocks of the hand-held test meter of FIG. 1;
FIGs. 3A is simplified electrical schematic diagrams, which, taken together with FIGs. 3B-1 and 3B-2, depict a first-time-on (FTO) electrical circuit block as can be employed in embodiments of the present invention;
FIG. 3B is a is a diagram depicting the manner in which the partial simplified electrical schematic diagrams of FIGs. 3B-1 and 3B-2 are arranged;

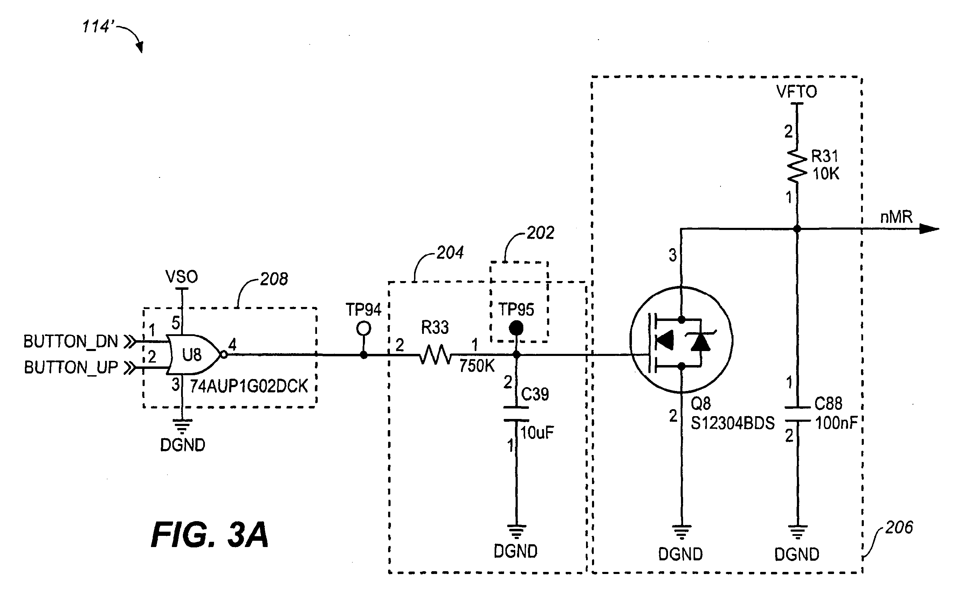

FIGs. 3B-1 and 3B-2 are simplified electrical schematic diagrams, which, taken together with FIG. 3A, depict a first-time-on (FTO) electrical circuit block as can be employed in embodiments of the present invention;

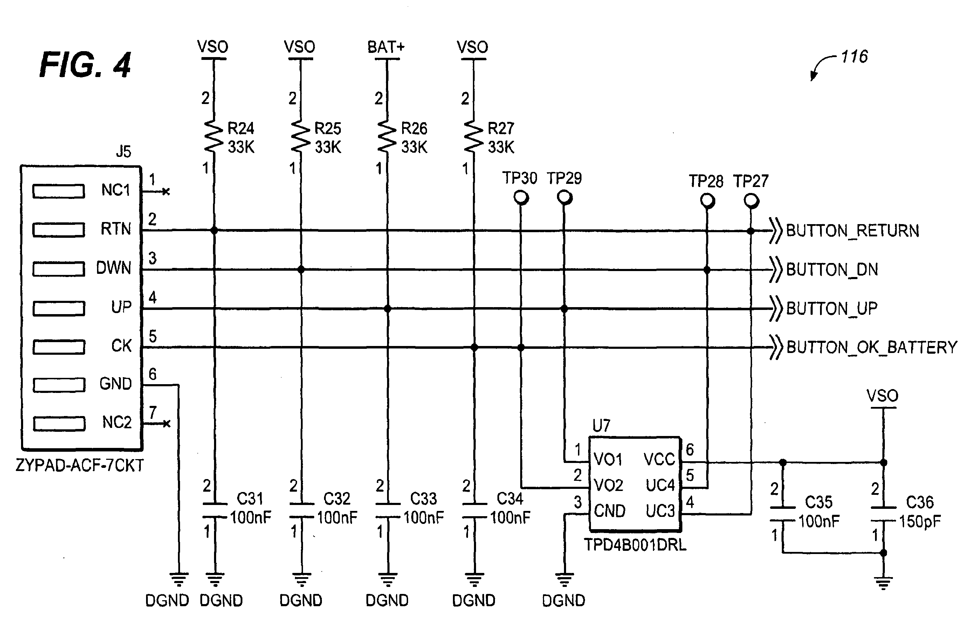

FIG. 4 is a simplified electrical schematic of a buttons electrical circuit block as can be employed in embodiments of the present invention; and

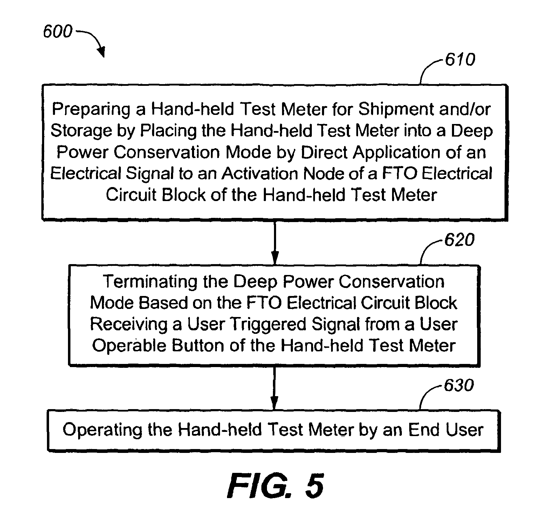

FIG. 5 is a flow diagram depicting stages in a method for employing a hand-held test meter according to an embodiment of the present invention.

### DETAILED DESCRIPTION OF ILLUSTRATIVE EMBODIMENTS

The following detailed description should be read with reference to the drawings, in which like elements in different drawings are identically numbered. The drawings, which are not necessarily to scale, depict exemplary embodiments for the purpose of explanation only and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein.

In general, hand-held test meters for use with an analytical test strip (e.g., an electrochemical-based analytical test strip) in the determination of an analyte (such as glucose) in a bodily fluid sample (for example, a whole blood sample) according to embodiments of the present invention include a housing, a buttons electrical circuit block, at least one user operable button in operable communication with the buttons electrical circuit block, and a first-time-on (FTO) electrical circuit block. In such hand-held test meters, the FTO electrical circuit block is disposed within the housing and includes an activation node. In addition, the FTO electrical circuit block is configured to place the hand-held test meter into a deep power conservation mode upon the direct application of an electrical signal to the activation node by an external device (e.g., a manufacturing tester) and to terminate the deep power conservation mode and place the hand-held test meter into a normal operating mode upon receiving a predetermined user triggered signal from the at least one user operable button.

Hand-held test meters according to embodiments of the present invention are beneficial in that, for example, the deep power conservation mode cannot be inadvertently activated by an end user (i.e., a health care professional demonstrating the hand-held test meter or a patient operating the hand-held test meter) as it requires the direct application of an electrical signal (such as an applied voltage from a manufacturing tester) to an activation node that is disposed within the hand-held test meter's housing (i.e., an internal activation node, also referred to as a test point). Such an internal activation node is not reasonably accessible to an end-user nor does an end-user typically have an external device that could apply the required electrical signal for activating the deep power conservation mode. Since the predetermined user triggered signal can be generated by an end-user's normal operation of the hand-held test meter including, for example, simply turning on (activating) the hand-held test meter by pushing an appropriate hand-held test meter button, termination of the deep power conservation mode is simple, intuitive and requires no dedicated actions on the part of an end user. Moreover, the deep power conservation mode enables shipment and prolonged storage of the hand-held test meter with a sealed rechargeable battery in a charged state without deleterious loss of charge. The hand-held meter is, therefore, ready for immediate operation (for example, an out-of-the-box test and demonstration) once the deep power conservation mode is terminated.

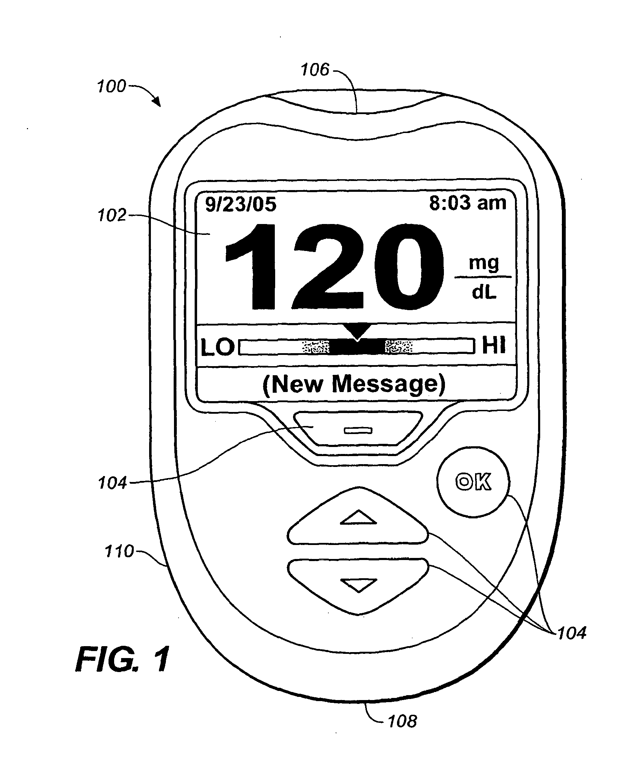

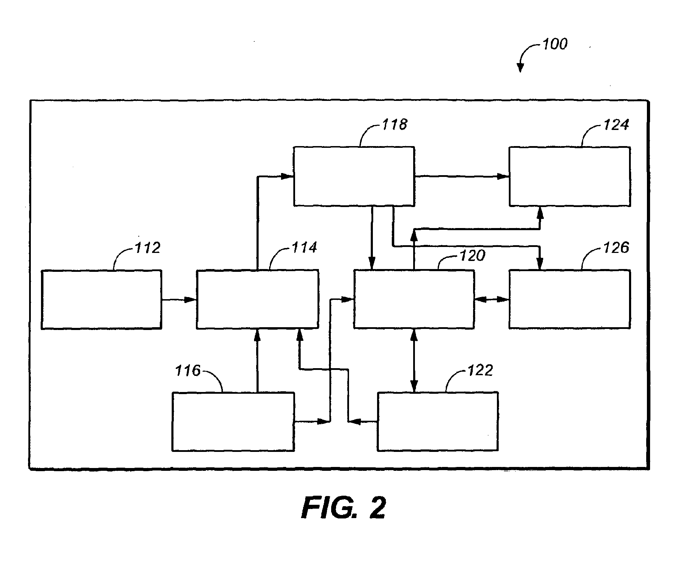

FIG. 1 is a simplified top view depiction of a hand-held test meter 100 with a deep power conservation circuitry block according to an embodiment of the present invention. FIG. 2 is a simplified block diagram of various blocks of the hand-held test meter 100.

Once one skilled in the art is apprised of the present disclosure, he will recognize that an example of a hand-held test meter that can be readily modified as a hand-hand test meter according to the present invention is the commercially available OneTouch^{®} Ultra^{®} 2 glucose meter from LifeScan Inc. (Milpitas, California). Additional examples of hand-held test meters that can also be modified are found in U.S. Patent Application Publications No's. 2007/0084734 (published on April 19, 2007) and 2007/0087397 (published on April 19, 2007) and in International Publication Number WO2010/049669 (published on May 6, 2010).

Hand-held test meter 100 includes a display 102, a plurality of user interface buttons 104, a strip port connector 106, a USB interface 108, and a housing 110 (see FIG. 1). Referring to FIG. 2 in particular, hand-held test meter 100 also includes a battery 112, a first-time-on (FTO) electrical circuit block 114, a buttons electrical circuit block 116, a power supply circuitry block 118, a microcontroller block 120, a communications port block 122, a display control block 124, a memory block 126 and other electronic components (not shown) for applying a test voltage to analytical test strip (not shown), and also for measuring an electrochemical response (e.g., plurality of test current values) and determining an analyte based on the electrochemical response. To simplify the current descriptions, the figures do not depict all such electronic circuitry.

Display 102 can be, for example, a liquid crystal display or a bi-stable display configured to show a screen image. An example of a screen image may include a glucose concentration, a date and time, an error message, and a user interface for instructing an end user how to perform a test.

Strip port connector 106 is configured to operatively interface with the analytical test strip (not depicted in the figures) such as an electrochemical-based analytical test strip configured for the determination of glucose in a whole blood sample. Therefore, the analytical test strip is configured for operative insertion into strip port connector 106. The analytical test strip can be any suitable analytical test strip including an electrochemical-based analytical test strip such as the commercially available OneTouch^{®} Ultra^{®} glucose test strip from LifeScan Inc. (Milpitas, California). Examples of analytical test strips can be found in U.S. Patent No's. 5,708,247; 5,951,836; 6,241,862; 6,284,125; 6,413,410; 6,733,655; 7,112,265; 7,241,265; and 7,250,105.

USB Interface 108 can be any suitable interface known to one skilled in the art. Moreover, USB interface 108 can configured such that battery 112 of hand-held test meter 100 is recharged via USB interface 108 using, for example, recharging techniques that are well known to those of skill in the art. USB Interface 108 is essentially a passive component that is configured to power and provide a data line to communications port block 122 of hand-held test meter 100.

Once an analytical test strip is interfaced with hand-held test meter 100, or prior thereto, a bodily fluid sample (e.g., a whole blood sample) is dosed into a sample-receiving chamber of the analytical test strip. The analytical test strip can include enzymatic reagents that selectively and quantitatively transforms an analyte into another predetermined chemical form. For example, the analytical test strip can include an enzymatic reagent with ferricyanide and glucose oxidase so that glucose can be physically transformed into an oxidized form.

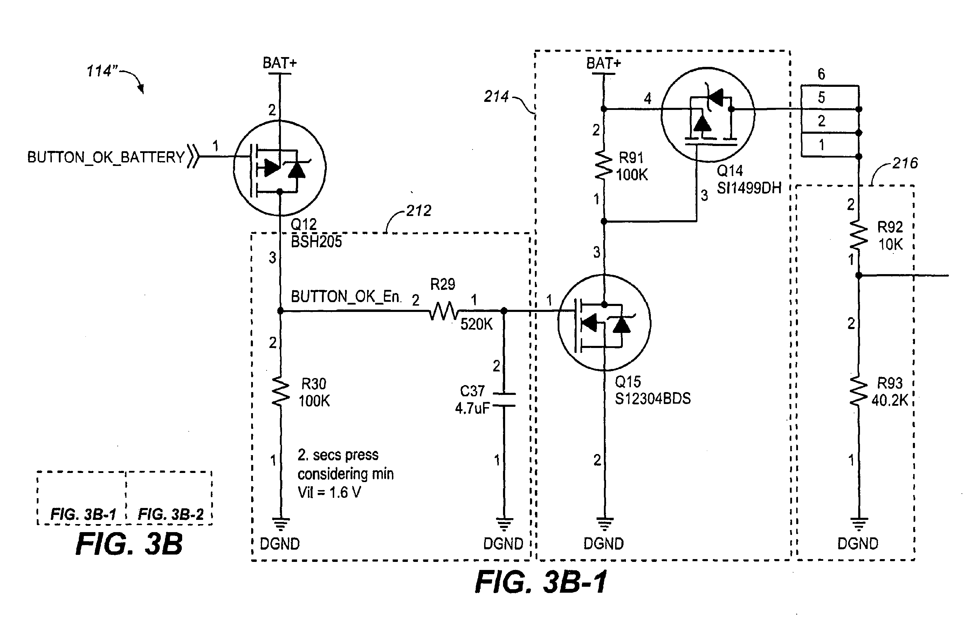

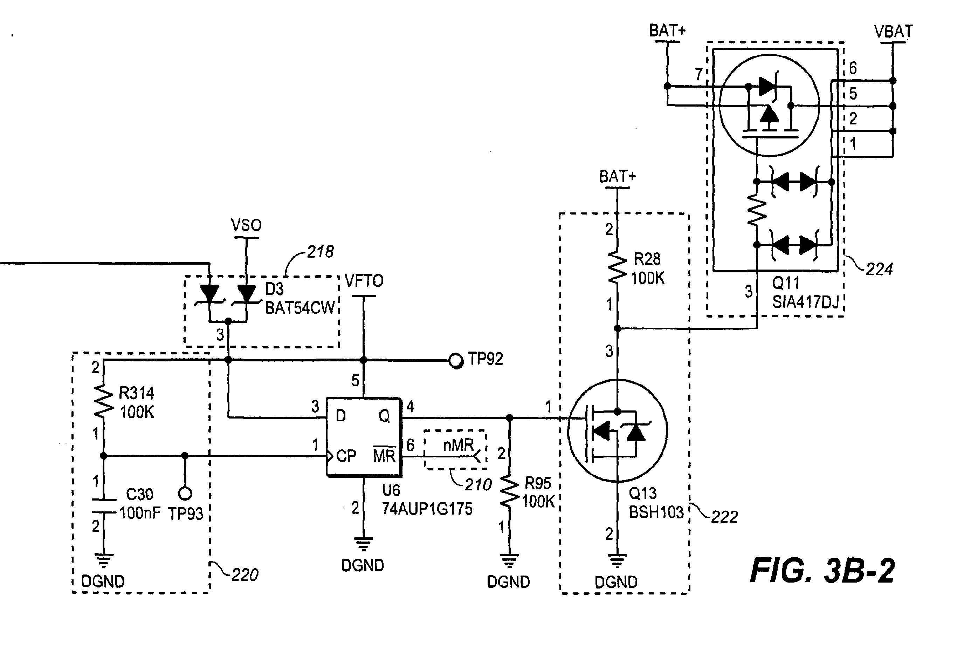

Battery 112 can be any suitable battery including, for example, a rechargeable battery permanently sealed within housing 100. Power supply circuitry block 118 includes, for example, Low Drop-out Regulator (LDO) and voltage regulation circuits well known to those skilled in the art. FTO electrical circuit block 114 and buttons electrical circuit block 116 are described in detail below with respect to FIGs. 3A, 3B and 4. Memory 126 block of hand-held test meter 100 includes a suitable algorithm that determines an analyte based on the electrochemical response of analytical test strip.

FIGs. 3A, 3B-1 and 3B-2 are simplified electrical schematic diagrams which, taken together, depict a first-time-on (FTO) electrical circuit block 114 as can be employed in embodiments of the present invention. Since FIG. 3A, 3B-1 and 3B-2 must be taken together to yield a FTO electrical circuit block 114, portions thereof are labeled as 114' (i.e., FIG. 3A) and 114" (i.e., the combination of FIGs. 3B-1 and 3B-2). FIG. 4 is a simplified electrical schematic of a buttons electrical circuit block 116 as can be employed in embodiments of the present invention.

FTO circuit block 114 is configured to place hand-held test meter 100 into a deep power conservation mode (also referred to as a deep sleep mode) only upon the direct application of an electrical signal to the activation node by an external device. The external device can be, for example, a manufacturing tester that is also employed to test the hand-held meter's functionality during manufacturing and prior to shipment to storage. Alternatively as an optional configuration, FTO electrical circuit block 114 can be configured to also place hand-held test meter 100 into a deep power conservation mode upon a user simultaneously pressing an "up" button and a "down" button of the hand-held test meter as explained further below.

FTO electrical circuit block 114 is also configured to terminate the deep power conservation mode and place hand-held test meter 100 into a normal operating mode upon receiving a predetermined user triggered signal from at least one user operable button.

The predetermined signal can be generated by buttons electrical circuit block 114, for example, by an end user pushing the OK button depicted in FIG. 1 for at least 2 seconds. FTO electrical circuit block 114 is also configured to terminate the deep power conservation mode and place hand-held test meter 100 into a normal operating mode upon attachment of a cable (such as a USB cable) to USB interface 108 or the attachment of a power supply cable to hand-held test meter 100 via USB Interface 108.

In the deep power conservation mode, hand-held test meter 100 consumes less than approximately 15 nA of power as power is only being consumed by battery 112 itself through any naturally occurring battery discharge mechanism and momentarily by the buttons electrical circuit block upon pressing of a button and not be any other blocks of the hand-held test meter (such as the FTO electrical circuit block, power supply block, microcontroller block, display control block communications port block and memory block).

Referring to FIGs, 3A, 3B and 4, the operation of FTO electrical circuit block 114 will now be described in more detail. For the purposes of such descriptive detail, FTO electrical circuit block 114 is partitioned into various sub-blocks 202, 204, 206, 208, 210, 212, 214, 216, 218, 220, 222 and 224 delineated by dashed lines in FIGs. 3A and 3B. One skilled in the art will recognize that such sub-blocks are for descriptive purposes only and that a FTO electrical circuit block employed in embodiments of the present invention can take a form that differs in detail from that of FIGs. 3A, 3B-1 and 3B-2.

Sub-block 202 of FTO electrical circuit block 114 is configured as the activation node that places hand-held test meter 100 into the deep power conservation mode when an electrical activation signal is applied thereto. This activation node is also labeled TP95 in FIG. 3A. Sub-block 204 is configured to provide an approximately 6 second time constant and, thereby, avoid accidental entry into the deep power conservation mode due to spurious signals.

Sub-block 206 is configured to translate an active high signal from sub-block 204 into an active low signal. The active low signal (labeled nMR on the right-hand side of FIG. 3A) is communicated to the remainder of FTO electrical circuit block 114 depicted in FIGs. 3B-1 and 3B-2. Sub-block 208 is an optional block configured to place hand-held test meter into the deep power conservation mode by a user simultaneously pushing both an "up" button and a "down" button of the hand-held test meter. Sub-block 208 is optional in that its absence can be beneficial as it would result in a hand-held test meter 100 that can only be placed into a deep sleep mode via sub-block 206, which is not user accessible. This prevents a user from accidentally activating the deep power conservation mode. However, if desired to provide for user activation of the deep power conservation mode by the pressing of two buttons simultaneously and, thus, likely not accidentally, sub-block 208 can be employed.

Sub-block 210 (see FIG. 3B-2) of FTO electrical circuit block 114 is configured to receive the nMR signal from sub-block 206 and communicate the signal to the remainder of FTO electrical circuit block 114 depicted in FIGs. 3B-1 and 3B-2. Sub-block 212 is configured to provide an approximately 2 second time constant before the deep power conservation mode is terminated and hand-held test meter 100 is placed into a normal operating mode upon receiving a predetermined user triggered signal (i.e., signal BUTTON_OK_BATTERY in FIG. 3B) from at least one user operable button.(see FIG. 4)

Sub-block 214 is configured to decouple the approximately 2 second time constant of sub-block 212 from the remainder of FTO electrical circuit block 114. Sub-block 216 is configured to lower the voltage level of battery 112 (for example, 4.2 volts) to suitable for use in the remainder of FTO electrical circuit block 114 (such as a 3.3V compliant voltage for flip-flop circuit U6 of FIG. 3B-2).

Sub-block 218 includes a pair of Schottky diodes and is configured to power-OR flip-flop U6. Any suitable Schottky diode can be employed including those commercially available from On Semiconductor. Flip-flop U6 can be any suitable flip-flop including, for example, a commercially available low power flip-flop from NXP as part number 74AUP1G175. Such a configuration enables FTO electrical circuit block 114 to provide for hand-held test meter 100 to terminate the deep power conservation mode upon either receipt of a predetermined user triggered signal or when the hand-held test meter receives power via USB interface 108 and communications port block 122. In this regard, it should be noted that hand-held test meter 100 is configured such that connecting a powered USB cable to USB interface 108 results in the powering of VSO, which is connected to flip-flop U6 via sub-block 218 (see FIG. 3B-2). This results in the switching on of Q11 (sub-block 224), which provides power to an LDO circuit of power supply block 118, which in turn powers microcontroller block 120.

Sub-block 220 is configured to trigger flip-flop U6 to change its output only when the power is set to a predetermined level. Sub-block 222 is configured to convert an active high signal to an active low signal. Sub-block 224 is configured connect battery 112 to an LDO circuit of power supply circuitry block 118.

In the deep power conservation mode, no power is consumed by the FTO electrical circuit block 114 or other circuit blocks of hand-held test meter 100 other than buttons electrical circuit block 116 in the event a button is pushed. Buttons electrical circuit block 116 is configured to only consume power when a button is pressed, typically for a duration of milliseconds to a few seconds (i.e., momentarily) to generate the predetermined user generated signal, Buttons electrical circuit block 116, therefore, only consumes an insignificant amount of power. The only notable power consumption in the deep power conservation mode is that associated with natural self-discharge of battery 112, and any battery protection circuit included in battery 112. During use, FTO electrical circuit block 114 is only powered in its entirety for the few seconds required to terminate the deep power conservation mode by electrically connecting battery 112 to power supply circuitry block 118. Once the deep power conservation mode is terminated, only flip-flop U6 and resistors R95 and R28 of FTO electrical circuit block 114 consume power.

Solid state switch Q11 (of sub-block 224) is employed to connect and to disconnect the power from battery 112. Once hand-held test meter 100 is placed into the deep power conservation mode or the normal operating mode, flip-flop U6 maintains the hand-held test meter in that state until an event occurs that changes the state. As explained above, in the embodiment of FIGs. 3A, 3B-1 and 3B-2, such an event is one of (i) receiving a predetermined user triggered signal (i.e., BUTTON_OK_BATTERY) from at least one user operable button for a duration exceeding 2 seconds; (ii) provision of power (VSO) via USB interface 108; and (iii) direct application of an electrical signal to the activation node (sub-block 202) by an external device. Flip-flop U6 stores the mode information in the status of output pin Q of flip-flop U6. If desired, FTO electrical circuit block 114 can be optionally configured to sense the insertion of a test strip into strip port connector 106 using, for example, sensing techniques known to one skilled in the art once apprised of the present disclosure. The sensing of test strip insertion into the hand held test meter can then be used to terminate a deep power conservation mode.

When a negative nMR signal is sensed by flip-flop U6, pin Q goes low. This low signal is converted into a high signal by sub-block 222 and switch Q11 is opened, thus placing hand-held test meter 100 into the deep power conservation mode. Such a negative nMR signal is obtained with a high signal on TP95 that closes component Q8 of sub-block 206, thereby connecting nMR to ground.

Upon a predetermined user triggered signal (i.e., BUTTON_OK_BATTERY) from at least one user operable button for a duration exceeding 2 seconds or provision of power (VSO) via USB interface 108, pin Q goes high. Sub-block 222 (a level shifter), translates this high signal to a low signal that closes switch Q11, thus terminating the deep power conservation mode.

FIG. 5 is a flow diagram depicting stages in a method 600 for operating a hand-held test meter configured for the determination of an analyte (such as glucose) in a bodily fluid sample (e.g., a whole blood sample) according to an embodiment of the present invention. Method 600 includes preparing the hand-held test meter for at least one of storage and shipment prior to end user operation of the hand-held test meter (see step 610 of FIG. 5). The preparation is accomplished by placing the hand-held test meter into a deep power conservation mode via the direct application of an electrical signal to an activation node of a first time on (FTO) electrical circuit block of the hand-held test meter by an external device (for example, a manufacturing tester employed in a manufacturing process for the hand-held test meter).

Method 600 also includes, at step 620, terminating the deep power conservation mode and placing the hand-held test meter into a normal operating mode based on the FTO electrical circuit block receiving a predetermined user triggered signal from a user operable button of the hand-held test meter, and subsequently at step 630 operating of the hand-held test meter by an end user.

In methods according to embodiments of the present invention, the hand-held test meter can be, for example, shipped from a hand-held test meter manufacturing site following the preparing step and prior to the terminating step. In addition, the hand-held test meter can, if desired, be stored following the preparing step and prior to the terminating step. Since the preparing step has placed the hand-held test meter into a deep power conservation mode, such shipping and storage can occur over relatively long durations without complete discharge of a battery included in the hand-held test meter.

Methods according to embodiments of the present invention can, if desired, also include the steps of (i) applying a bodily fluid sample to an electrochemical-based analytical test strip; (ii) measuring an electrochemical response of the electrochemical-based analytical test strip using the hand-held test meter; and (iii) determining the analyte based on the measured electrochemical response.

Once apprised of the present disclosure, one skilled in the art will recognize that method 600 can be readily modified to incorporate any of the techniques, benefits and characteristics of hand-held test meters according to embodiments of the present invention and described herein.

## Claims

1. A hand-held test meter (100) for use with an analytical test strip in the determination of an analyte in a bodily fluid sample, the hand-held test meter comprising:
a housing (110);
a buttons electrical circuit block (116);
at least one user operable button (104) in operable communication with the buttons electrical circuit block; and
a first-time-on (FTO) electrical circuit block (114) disposed within the housing, the FTO electrical circuit block including an activation node (TP95), and
wherein the FTO electrical circuit block is configured to place the hand-held test meter (100) into a deep power conservation mode upon the direct application of an electrical signal to the activation node by an external device; and
wherein the FTO electrical circuit block (114) is configured to terminate the deep power conservation mode and place the hand-held test meter (100) into a normal operating mode upon receiving a predetermined user triggered signal from the at least one user operable button (104), wherein the FTO electrical circuit block is further configured to terminate the deep power conservation mode and place the hand-held test meter (100) into a normal operating mode upon insertion of a test strip into the hand-held meter.

2. The hand-held test meter (100) of claim 1 wherein the FTO electrical circuit block (114) is further configured to permanently terminate the deep power conservation mode upon receiving a predetermined user triggered signal from the at least one user operable button (104).

3. The hand-held test meter (100) of claim 1 further including:
a rechargeable battery (112) disposed within the housing.

4. The hand-held test meter (100) of claim 3 wherein power from the rechargeable battery is momentarily available to the buttons electrical circuit block for generation of the predetermined user generated signal when the hand-held test meter is in the deep power conservation mode.

5. The hand-held test meter (100) of claim 3 wherein the rechargeable battery (112) is permanently sealed within the housing.

6. The hand-held test meter (100) of claim 1 further including a communications port block.

7. The hand-held test meter (100) of claim 6 wherein the FTO electrical circuit block is further configured to terminate the deep power conservation mode and place the hand-held test meter into a normal operating mode upon attachment of a communications cable to the communications port block or a power cable to the hand-held test meter.

8. The hand-held test meter (100) of claim 1 wherein the hand-held test meter (100) consumes less than approximately 15 nA of power in the deep power conservation mode.

9. The hand-held test meter (100) of claim 1 wherein the hand-held test meter is configured for the determination of glucose in a whole blood sample.

10. A method (600) for employing a hand-held test meter (100) configured for the determination of an analyte in a bodily fluid sample, the method comprising:
preparing a hand-held test meter for at least one of storage and shipment prior to end user operation of the hand-held test meter by placing the hand-held test meter into a deep power conservation mode via the direct application of an electrical signal to an activation node of a first time on (FTO) electrical circuit block of the hand-held test meter by an external device;
terminating the deep power conservation mode and placing the hand-held test meter into a normal operating mode based on the FTO electrical circuit block receiving a predetermined user triggered signal upon insertion of a test strip into the hand-held meter; and
operating of the hand-held test meter by an end user.

11. The method (600) of claim 10 wherein the external device is a manufacturing tester employed in a manufacturing process for the hand-held test meter (100).

12. The method (600) of claim 10 further including the step of shipping the hand-held test meter (100) from a hand-held test meter manufacturing site following the preparing step (610) and prior to the terminating step (620).

13. The method (600) of claim 10 further including the step of storing the hand-held test meter (100) following the preparing step (610) and prior to the terminating step (620).

14. The method (600) of claim 11 wherein the terminating step (620) occurs based on the FTO electrical circuit block receiving a predetermined user triggered signal from a user operable button of the hand-held test meter (100); or
sensing the attachment of a communications cable to the hand-held test meter (100); or sensing the attachment of a power cable to the hand-held test meter.

15. The method (600) of claim 10 wherein the preparing step is via the direct application of a voltage signal to the activation node (TP95).

16. The method (600) of claim 10 further including:
applying a bodily fluid sample to an electrochemical-based analytical test strip;
measuring an electrochemical response of the electrochemical-based analytical test strip using the hand-held test meter (100); and
determining the analyte based on the measured electrochemical response.

17. The method (600) of claim 16 wherein the bodily fluid sample is a whole blood sample and the analyte is glucose.

## Patentansprüche

1. Tragbares Messgerät (100) zur Verwendung mit einem analytischen Teststreifen bei der Bestimmung eines Analyts in einer Körperfluidprobe, wobei das tragbare Messgerät Folgendes umfasst:
ein Gehäuse (110);
einen Tastenbereich für den Stromkreis (116);
wenigstens eine vom Anwender bedienbare Taste (104), die mit dem Tastenbereich für den Stromkreis in betriebstechnischer Kommunikation ist; und
einen Bereich für den Stromkreis für das erstmalige Einschalten (FTO) (114), der in dem Gehäuse angeordnet ist, wobei der FTO-Bereich für den Stromkreis einen Aktivierungsknoten (TP95) umfasst, und
wobei der FTO-Bereich für den Stromkreis so ausgelegt ist, dass das tragbare Messgerät (100) bei dem direkten Anlegen eines elektrischen Signals an den Aktivierungsknoten durch eine externe Vorrichtung in eine Energiesparbetriebsart versetzt wird; und
wobei der FTO-Bereich für den Stromkreis (114) ausgelegt ist, beim Empfangen eines vom Anwender ausgelösten vorgegebenen Signals von der wenigstens einen durch den Anwender bedienbaren Taste (104) die Energiesparbetriebsart zu beenden und das tragbare Messgerät (100) in einen normalen Betriebszustand zu versetzen, wobei der FTO-Bereich für den Stromkreis ferner so ausgelegt ist, beim Einsetzen eines Teststreifens in das tragbare Messgerät die Energiesparbetriebsart zu beenden und das tragbare Messgerät (100) in einen normalen Betriebszustand zu versetzen.

2. Tragbares Messgerät (100) nach Anspruch 1, wobei der FTO-Bereich für den Stromkreis (114) ferner ausgelegt ist, die Energiesparbetriebsart beim Empfangen eines vom Anwender ausgelösten, vorgegebenen Signals von der wenigstens einen vom Anwender bedienbaren Taste (104) dauerhaft zu beenden.

3. Tragbares Messgerät (100) nach Anspruch 1, das ferner Folgendes umfasst:
eine in dem Gehäuse angeordnete wiederaufladbare Batterie (112).

4. Tragbares Messgerät (100) nach Anspruch 3, wobei Leistung von der wiederaufladbaren Batterie zeitweise für den Tastenbereich für den Stromkreis zur Erzeugung des vom Anwender erzeugten, vorgegebenen Signals verfügbar ist, wenn sich das tragbare Messgerät in der Energiesparbetriebsart befindet.

5. Tragbares Messgerät (100) nach Anspruch 3, wobei die wiederaufladbare Batterie (112) dauerhaft in dem Gehäuse eingeschlossen ist.

6. Tragbares Messgerät (100) nach Anspruch 1, das ferner einen Kommunikationsanschlussbereich umfasst.

7. Tragbares Messgerät (100) nach Anspruch 6, wobei der FTO-Bereich für den Stromkreis ferner ausgelegt ist, beim Befestigen eines Kommunikationskabels an dem Kommunikationsanschlussbereich oder eines Stromkabels an dem tragbaren Messgerät die Energiesparbetriebsart zu beenden und das tragbare Messgerät in einen normalen Betriebszustand zu versetzen.

8. Tragbares Messgerät (100) nach Anspruch 1, wobei das tragbare Messgerät (100) in der Energiesparbetriebsart weniger als etwa 15 nA Strom verbraucht.

9. Tragbares Messgerät (100) nach Anspruch 1, wobei das tragbare Messgerät für die Bestimmung von Glukose in einer Vollblutprobe ausgelegt ist.

10. Verfahren (600) zur Nutzung eines tragbaren Messgeräts (100), das zur Bestimmung eines Analyts in einer Körperfluidprobe ausgelegt ist, wobei das Verfahren folgende Schritte umfasst:
Vorbereiten eines tragbaren Messgeräts wenigstens für die Lagerung oder den Versand vor dem Einsatz beim Endanwender des tragbaren Messgeräts durch Versetzen des tragbaren Messgeräts in eine Energiesparbetriebsart mittels des direkten Anlegens eines elektrischen Signals an einem Aktivierungsknoten eines FTO-Bereichs für den Stromkreis des tragbaren Messgeräts durch eine externe Vorrichtung;
Beenden der Energiesparbetriebsart und Versetzen des tragbaren Messgeräts in einen normalen Betriebszustand basierend auf dem FTO-Bereich für den Stromkreis, der ein vom Anwender ausgelöstes, vorgegebenes Signal beim Einsetzen eines Teststreifens in das tragbare Messgerät empfängt; und
Betreiben des tragbaren Messgeräts durch einen Endanwender.

11. Verfahren (600) nach Anspruch 10, wobei die externe Vorrichtung ein Herstellungsprüfgerät ist, das in einem Herstellungsverfahren für das tragbare Messgerät (100) genutzt wird.

12. Verfahren (600) nach Anspruch 10, das ferner den Schritt des Versendens des tragbaren Messgeräts (100) von einem Herstellungsstandort des tragbaren Messgeräts nach dem Vorbereitungsschritt (610) und vor dem Beendigungsschritt (620) umfasst.

13. Verfahren (600) nach Anspruch 10, das ferner den Schritt des Lagerns des tragbaren Messgeräts (100) nach dem Vorbereitungsschritt (610) und vor dem Beendigungsschritt (620) umfasst.

14. Verfahren (600) nach Anspruch 11, wobei der Beendigungsschritt (620) basierend auf dem FTO-Bereich für den Stromkreis erfolgt, der ein vom Anwender ausgelöstes, vorgegebenes Signal von einer durch den Anwender bedienbaren Taste des tragbaren Messgeräts (100) erhält; oder
Erkennen der Befestigung eines Kommunikationskabels an dem Tragbares Messgerät (100); oder
Erkennen der Befestigung eines Stromkabels an dem Tragbares Messgerät.

15. Verfahren (600) nach Anspruch 10, wobei der Vorbereitungsschritt mittels des direkten Anlegens eines Spannungssignals an den Aktivierungsknoten (TP95) erfolgt.

16. Verfahren (600) nach Anspruch 10, das ferner die folgenden Schritte umfasst:
Aufbringen einer Körperfluidprobe auf einen auf Elektrochemie basierenden analytischen Teststreifen;
Messen einer elektrochemischen Antwort des auf Elektrochemie basierenden analytischen Teststreifens unter Verwendung des tragbaren Messgeräts (100); und
Bestimmen des Analyts anhand der gemessenen elektrochemischen Antwort.

17. Verfahren (600) nach Anspruch 16, wobei die Körperfluidprobe eine Vollblutprobe ist und der Analyt Glukose ist.

## Revendications

1. Contrôleur à main (100) pour une utilisation avec une bande de test analytique pour déterminer un analyte dans un échantillon de fluide corporel, le contrôleur à main comprenant :
un boîtier (110) ;
un bloc de circuit électrique à boutons (116) ;
au moins un bouton actionnable par un utilisateur (104) en communication fonctionnelle avec le bloc de circuit électrique à boutons ; et
un bloc de circuit électrique de première mise en service (FTO) (114) disposé dans le boîtier, le bloc de circuit électrique FTO comprenant un noeud d'activation (TP95), et
où le bloc de circuit électrique FTO est configuré pour placer le contrôleur à main (100) en mode d'économie d'énergie profond lors de l'application directe d'un signal électrique sur le noeud d'activation par un dispositif externe ; et
où le bloc de circuit électrique FTO (114) est configuré pour mettre fin au mode d'économie d'énergie profond et placer le contrôleur à main (100) en mode de fonctionnement normal à réception d'un signal prédéterminé déclenché par un utilisateur depuis l'au moins un bouton actionnable par un utilisateur (104), où le bloc de circuit électrique FTO est en outre configuré pour mettre fin au mode d'économie d'énergie profond et placer le contrôleur à main (100) en mode de fonctionnement normal à l'insertion d'une bande de test dans le compteur à main.

2. Contrôleur à main (100) selon la revendication 1, dans lequel le bloc de circuit électrique FTO (114) est en outre configuré pour mettre fin définitivement au mode d'économie d'énergie profond à réception d'un signal prédéterminé déclenché par un utilisateur depuis l'au moins un bouton actionnable par un utilisateur (104).

3. Contrôleur à main (100) selon la revendication 1, comprenant en outre :
une batterie rechargeable (112) disposée dans le boîtier.

4. Contrôleur à main (100) selon la revendication 3, dans lequel l'énergie provenant de la batterie rechargeable est momentanément disponible pour le bloc de circuit électrique à boutons pour générer le signal prédéterminé généré par un utilisateur lorsque le contrôleur à main se trouve en mode d'économie d'énergie profond.

5. Contrôleur à main (100) selon la revendication 3, dans lequel la batterie rechargeable (112) est scellée de manière permanente dans le boîtier.

6. Contrôleur à main (100) selon la revendication 1, comprenant en outre un bloc de port de communication.

7. Contrôleur à main (100) selon la revendication 6, dans lequel le bloc de circuit électrique FTO est en outre configuré pour mettre fin au mode d'économie d'énergie profond et pour placer le contrôleur à main en mode de fonctionnement normal lors de le branchement d'un câble de communication au bloc de port de communication ou d'un câble d'alimentation au contrôleur à main.

8. Contrôleur à main (100) selon la revendication 1 dans lequel le contrôleur à main (100) consomme moins d'approximativement 15 nA d'électricité en mode d'économie d'énergie profond.

9. Contrôleur à main (100) selon la revendication 1, dans lequel le contrôleur à main est configuré pour déterminer du glucose dans un échantillon de sang total.

10. Procédé (600) pour utiliser un contrôleur à main (100) configuré pour la détermination d'un analyte dans un échantillon de fluide corporel, le procédé comprenant les étapes suivantes :
préparer un contrôleur à main pour au moins un stockage et une livraison avant l'utilisation du contrôleur à main par un utilisateur final en plaçant le contrôleur à main en mode d'économie d'énergie profond via l'application directe d'un signal électrique sur un noeud d'activation d'un bloc de circuit électrique de première mise en service (FTO) du contrôleur à main par un dispositif externe ;
mettre fin au mode d'économie d'énergie profond et placer le contrôleur à main en mode de fonctionnement normal sur la base du bloc de circuit électrique FTO recevant un signal prédéterminé déclenché par un utilisateur à l'insertion d'une bande de test dans le contrôleur à main ; et
faire fonctionner le contrôleur à main par un utilisateur final.

11. Procédé (600) selon la revendication 10, dans lequel le dispositif externe est un contrôleur de fabrication utilisé dans un processus de fabrication pour le contrôleur à main (100).

12. Procédé (600) selon la revendication 10, comprenant en outre l'étape consistant à livrer le contrôleur à main (100) depuis un site de fabrication de contrôleur à main, suite à l'étape de préparation (610) et avant l'étape d'achèvement (620).

13. Procédé (600) selon la revendication 10, comprenant en outre l'étape consistant à stocker le contrôleur à main (100), suite à l'étape de préparation (610) et avant l'étape d'achèvement (620).

14. Procédé (600) selon la revendication 11, dans lequel l'étape d'achèvement (620) survient sur la base du bloc de circuit électrique FTO :
recevant un signal prédéterminé déclenché par un utilisateur depuis un bouton actionnable par un utilisateur du contrôleur à main (100) ; ou
détectant le branchement d'un câble de communication au contrôleur à main (100) ; ou
détectant le branchement d'un câble d'alimentation au contrôleur à main.

15. Procédé (600) selon la revendication 10, dans lequel l'étape de préparation se fait via l'application directe d'un signal de tension sur le noeud d'activation (TP95).

16. Procédé (600) selon la revendication 10, comprenant en outre les étapes suivantes :
appliquer un échantillon de fluide corporel sur une bande de test analytique à base électrochimique ;
mesurer une réponse électrochimique de la bande de test analytique à base électrochimique au moyen du contrôleur à main (100) ; et
déterminer l'analyte sur la base de la réponse électrochimique mesurée.

17. Procédé (600) selon la revendication 16, dans lequel l'échantillon de fluide corporel est un échantillon de sang total et l'analyte est du glucose.
